# EUROPEAN PATENT SPECIFICATION

(11) **EP 2 809 279 B1**
(45) Date of publication and mention of the grant of the patent: **30.03.2016**
(21) Application number: 13705529.9
(22) Date of filing: 25.01.2013
(51) Int. Cl.: A61F 5/441

(54) **GAS FLOW REGULATION DEVICE**
GASDURCHFLUSSREGELUNGSVORRICHTUNG
DISPOSITIF DE RÉGULATION DE FLUX DE GAZ

(30) Priority: 30.01.2012 GB 201201528
(43) Date of publication of application: 10.12.2014
(73) Proprietor: Salts Healthcare Limited, Birmingham B7 4AA (GB)
(72) Inventor: WILTSHIRE, Neil, Birmingham, West Midlands B7 4AA (GB)
(74) Representative: Ashton, Timothy
(86) International application number: PCT/GB2013/050166
(87) International publication number: WO 2013/114085

(56) References cited:
- EP-A1- 0 985 390
- US-A- 4 938 750
- US-A- 5 968 024

## Description

This invention relates to a gas flow regulation device for connection to a gas outlet from an ostomy appliance. More particularly, but not exclusively, this invention relates to a gas flow regulation device for connection to a gas outlet from a colostomy appliance.

An ostomy appliance is used for collecting human waste from a stoma, which is a surgically constructed tube of a user's digestive tract that protrudes through a user's skin. Different types of ostomy appliances are used depending on the type of stoma. If the stoma is from the small intestine (ileum) a drainable ostomy appliance is often preferred, because the waste collected is generally in liquid form and can easily be empted from the appliance. If the stoma is from the large intestine or colon, then a non-drainable ostomy appliance is preferred. This is because the collected waste is usually solid or semi-solid, and it is therefore not possible to easily empty the appliance.

A problem with ostomy appliances, particularly appliances which are not drainable and include a gas outlet (often in combination with a filter), is that 'pancaking' can occur during use. This is where side walls of the bag of the appliance stick together preventing waste from falling to the bottom of the bag. Waste thus collects in the upper region of the bag and can block gas outlets from the bag or even force the bag to become detached from the user.

EP0985390 discloses an ostomy pouch including a front wall provided with a comfort layer and a vent. An aperture cover layer is secured to the front wall through the comfort layer to define a chamber for a filter element and to provide a smooth surface to which can be applied an adhesive sticker. The cover layer includes a plurality of apertures, and the flow rate through the vent can be controlled by positioning the sticker to selectively block one or more of the apertures.

US4938750 discloses a member for an ostomy pouch in the form of an overfilm or molded housing affixed to the pouch wall having a plurality of isolated vent passages. A seperate filter assembly is situated in each of the passages. Adhesive coated covers are situated to obstruct the flow through all but a selected one of the passages.

According to a first aspect of the invention, we provide a gas flow regulation device for connection to a gas outlet of an ostomy appliance, the device including:
an inlet for communicating with the gas outlet of the ostomy appliance;
an outlet from which gas can exit the device;
means for closing the outlet from the device; and
a gas flow path between the inlet and the outlet,
wherein said means includes folding the gas flow path back on itself to permit the rate of gas flow through the device to be user selectable.

Further features of the invention are set out in claims 2 to 14 appended hereto.

According to a second aspect of the invention, we provide an ostomy appliance including a device according to the first aspect of the invention coupled to a gas outlet thereof.

Embodiments of the invention will now be described by way of example only with reference to the accompanying drawings, of which:-
Figure 1 is plan view of a first embodiment of a device in accordance with the present invention in a first condition;
Figure 2 is a sectional view, along the plan A-A, of the device of figure 1;
Figure 3 is plan view of the device of figure 1 in a second condition;
Figure 4 is side view of the device of figure 1 in a third condition;
Figure 5 is a side view of an ostomy appliance including the device of figure 1;
Figure 6 is plan view of a second embodiment of a device in accordance with the present invention in a first condition;
Figure 7 is a sectional view, along the plan A-A ,of the device of figure 6;
Figure 8 is plan view of the device of figure 1 in a second condition;
Figure 9 is side view of the device of figure 1 in a third condition;
Figure 10 is side view of the device of figure 1 in a fourth condition;
Figure 11 is a side view of an ostomy appliance including the device of figure 6;
Figure 12 is plan view of a third embodiment of a device in accordance with the present invention in a first condition;
Figure 13 a sectional view, along the plan A-A, of the device of figure 12;
Figure 14 is plan view of the device of figure 12 in a second condition;
Figure 15 a sectional view, along the plan B-B, of the device in the second condition;
Figure 16 is a side view of an ostomy appliance including the device of figure 12;
Figure 17 is plan view of a third embodiment of a device in accordance with the present invention in a first condition;
Figure 18 a sectional view, along the plan A-A, of the device of figure 17;
Figure 19 is plan view of the device of figure 17 in a second condition;
Figure 20 a sectional view, along the plan B-B, of the device in the second condition; and
Figure 21 is a side view of an ostomy appliance including the device of figure 17.

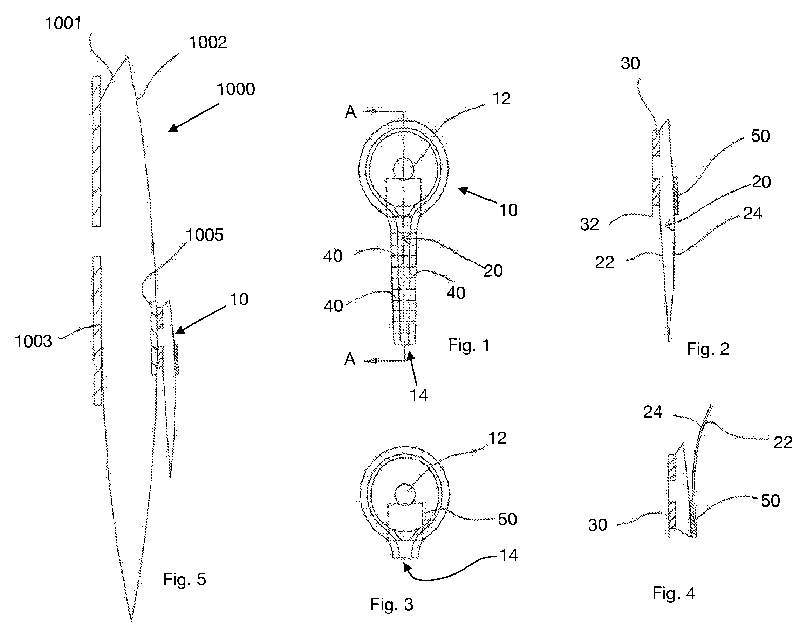

Referring firstly to figures 1 to 5 there is shown a first embodiment of a device 10 in accordance with the present invention. The device 10 is a gas flow regulation device for connection to a gas outlet of an ostomy appliance (1000, see fig. 5). The device 10 includes an inlet, in this case a generally circular aperture 12, for communicating with a gas outlet of the ostomy appliance 1000. As can be seen from figure 5 the ostomy appliance 1000 includes a pair of walls 1001, 1002, a hydrocolloid wafer 1003 and an outlet which is covered by a gas filter 1005. The inlet 12 communicates with the outlet from this filter 1005.

The device 10 includes an outlet 14 from which gas can exit the device and a gas flow path 20 which extends between the inlet 12 and the outlet 14. In the present example the device 10 includes a generally circular, in plan view, upper part which is connected to a generally elongate part of the gas flow path 20. In this example the cross sectional area of this part of the gas flow path 20 varies as it extends from the generally circular part towards the outlet 14. In more detail, the cross sectional area of this part of the gas flow path 20 gradually reduces, e.g. tapers, as it extends from the generally circular part towards the outlet 14.

The device 10 is manufactured from a pair of walls 22, 24, which are connected to each other at or near their peripheries. The walls 22, 24 should preferably be fully or virtually gas impermeable and are preferably connected to each other by heat welding or by an adhesive at or near their peripheries.

The device includes a means for fluid decoupling the inlet 12 to the gas outlet 1005. In this example such means is provided by an annular adhesive pad 30 which is covered by a removable cover member 32. As shown in figure 5 the adhesive pad 30 adheres to the wall 1002 of the ostomy appliance 1000. It should be noted for all embodiments discussed herein that whilst an adhesive is provided for coupling the device to the ostomy appliance, many other forms of attachment means could be used. For example, a wall of the device could be heat welded or otherwise adhered to one of the walls of the ostomy appliance.

The device 10 as shown in figures 1 to 5 is configured such that a length of the gas flow path 20 is changeable by a user to alter the rate of flow of gas through the device 10. In more detail, one or more portions of the gas flow path 20 of the device 10 are selectably removable by a user to alter the rate of gas flow through the device 10. Referring specifically to figure 1, a surface of the wall 24 (or the wall 22) includes indicia for indicating to a user the removable portions of the device 10. In the present example the indicia 40 are dashed lines which extend laterally across the gas flow path 20, but the indicia could take the form of solid lines, for example. Whilst not shown on figure 1, the device 10 may include indicia for indicating to a user a length change of the gas flow path 20 which is required to achieve a desired flow rate through the device 10.

In order to reduce the length of the gas flow path 20, a user makes an incision across the gas flow path 20 at the desired indicia 40, for example, using a sharp implement such as a pair of scissors.

The device 10 includes a means for closing the outlet 14 from the device. In the present example such means is provided by an adhesive pad 50 against which the gas flow path 20 can be folded and held in order to block the gas flow path 20. The adhesive pad 50 is, prior to use, covered by an adhesive cover member. Referring specifically to figure 4, this shows the device 10 in a fully closed configuration with the elongate part of the gas flow 20 having been folded back on itself and adhered to the adhesive pad 50. In this configuration gas is inhibited from flowing through the device.

Figure 3 shows the fully open configuration where a user has removed nearly all of the elongate part of the gas flow path 20, thus opening up the opening 14 as much as possible. In this configuration maximal gas flow can be achieved through the device 10.

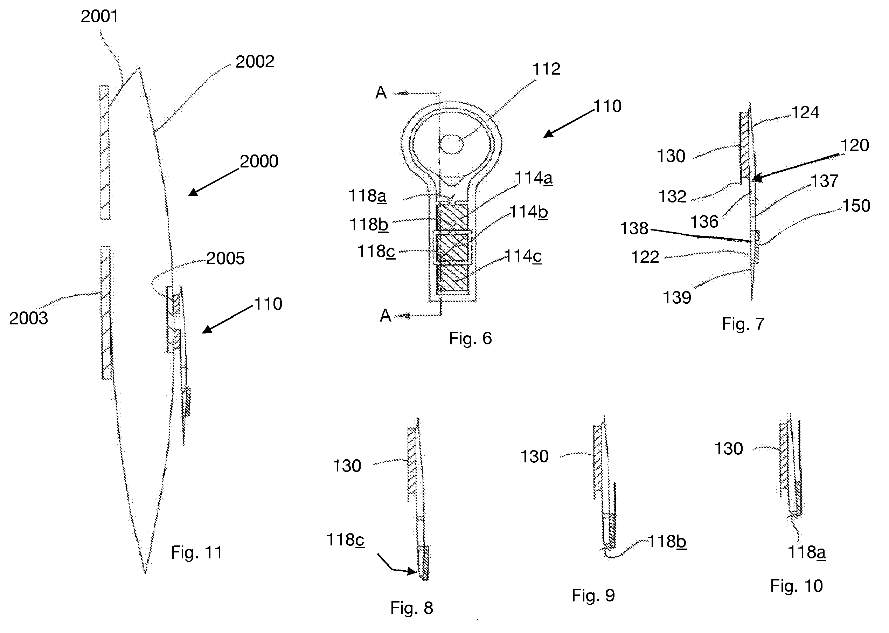

Referring now to figures 6 to 11 there is shown a second embodiment of a device 110 in accordance with the present invention. The device 110 shares a number of features with the first embodiment 10. Thus, features which are the same or perform the same function have been given the same reference number with the additional 100. The device 110 includes an inlet 112 for connection to a gas outlet 2005 of an ostomy appliance 2000. The device 110 includes, in this example, a plurality of outlets 114a, 114b, 114c which are in fluid communication with the inlet 112 and from which gas can exit the device 110.

In the present example the gas flow path 120 is divided into a plurality of chambers 136, 137, 138, 139. Chambers 137, 138, 139 include a respective outlet 114a, 114b, 114c for gas from the device 110. The chambers 136, 137, 138, 139 are connected by a number of openings 118a, 118b, 118c through which gas can flow from one chamber to another chamber. In the present example the device 110 is manufactured from a pair of walls 122, 124, which are connected to each other at or near their peripheries, for example by heat welding. In the present example the openings 118a, 118b, 118c are achieved by partially heat sealing across the gas flow path 120.

In the present example chambers 137, 138, 139 are in sequential gas communication with each other, but it should be appreciated that this not need be the case in order for the device to operate.

In the present example the outlets 114a, 114b, 114c are provided as gas permeable membranes which permit gas to pass therethrough. It should be appreciated, however, that the outlets 114a, 114b, 114c may be provided as apertures in one or both of the walls 122, 124 of the device 110 or they may be provided as a vented area in the walls 122, 124, such as by providing a plurality of later-cut apertures.

The device 110 includes means for closing one or more of the plurality of outlets 114a, 114b, 114c, thus permitting a user to select a desired rate of gas flow through the device 110. Closing of the outlets 114a, 114b, 114c, is achieved by folding the respective part of the gas flow path 120 back on itself with the fold line being about one of the openings 118a, 118b, 118c. In order to hold the device 110 in one of its folded configurations, an adhesive member 150 is provided connected to the wall 124 opposite the membrane 114b. Prior to use, the adhesive pad 150 is covered by a removable cover member.

Figures 8, 9 and 10 show three folded configurations of the device 110. In figure 8 the gas flow path 120 has been folded about the opening 118c. Thus gas can continue to flow into the inlet 112 and out from both of the membranes 114a, 114b. In figure 9 the gas flow path 120 has been folded about the opening 118b. Thus, gas can flow through the inlet 112 and out through the membrane 114a. In figure 10 the gas flow path 120 has been folded about the opening 118a, which means that gas cannot pass through the device as the gas flow path to all of the outlets 114a, 114b, 114c is blocked.

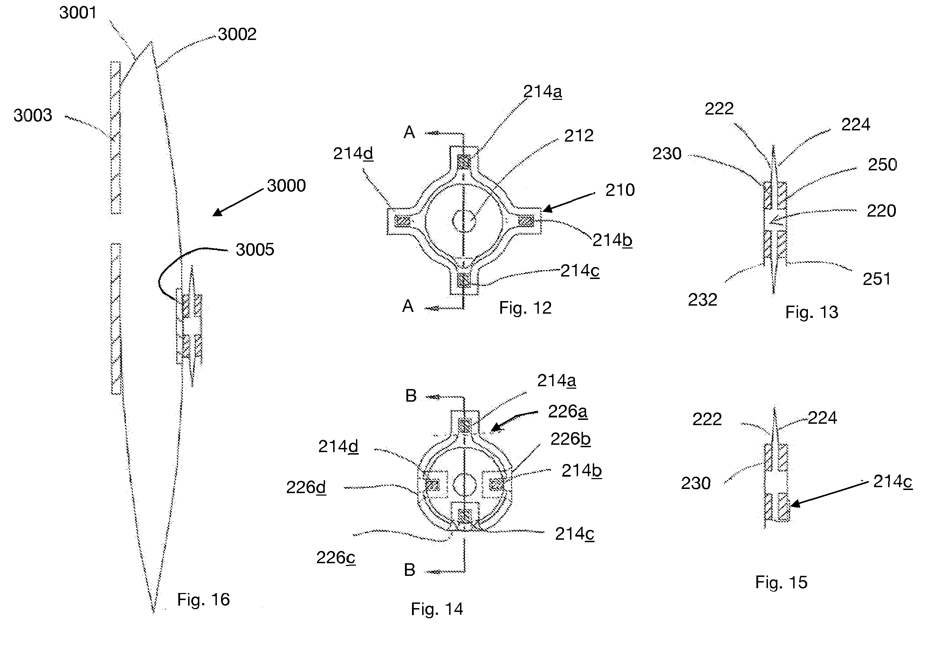

Referring now to figures 12 to 16 there is shown a third embodiment of a device 210 in accordance with the present invention. The device 210 includes a plurality of outlets 214a, 214b, 214c, 214d each of which communicates with the inlet 212 of the device. The device includes an adhesive pad 230 for connecting the device to ostomy appliance 3000, which is covered by a removable cover membrane 232 prior to application.

In the present example the outlets 214a, 214b, 214c, 214d are each provided as a gas permeable membrane in a wall 222 or 224 of the device 210. It should be appreciated, however, that the outlets 214a, 214b, 214c, 214d may be provided as apertures in one or both of the walls 222, 224 or as vented area, e.g. by providing a plurality of laser apertures in one or both of the walls 222, 224.

In the present example the size of each of the outlets 214a, 214b, 214c, 214c, 214d are substantially the same. Thus, in the present examples a cross sectional area of each of the outlets is substantially the same. It should be appreciated, however, that the outlets 214a, 214b, 214c, 214d may be sized differently from each other, to permit different flow rates of gas therethrough.

The device 210 permits a user to select a desired rate of gas flow through the device 210. This is achieved by selectively closing one or more of the outlets 214a, 214b, 214c, 214d. Closing of the outlets 214a, 214b, 214c, 214d is achieved by folding the respective outlet back on itself so that the gas flow path 220 thereto is blocked. In the present example means is provided for holding the outlet 214a, 214b, 214c, 214d in their closed configuration. Such means is provided by an adhesive pad 250 which is covered by a removable cover member 251. The adhesive pad is substantially identical to the adhesive pad 230.

In use one or more of the outlets are closed by folding the outlet about its respective opening 226a, 226b, 226c, 226d, thus blocking the flow of gas from the inlet 212 to that outlet. Figure 14 shows the configuration where three of the outlets 214b, 214c, 214d have been closed, thus permitting gas only to pass from the inlet 212 to the outlet 214a. It should be appreciated, of course, that any combination of the outlets 214a, 214b, 214c, 214d can be closed/open.

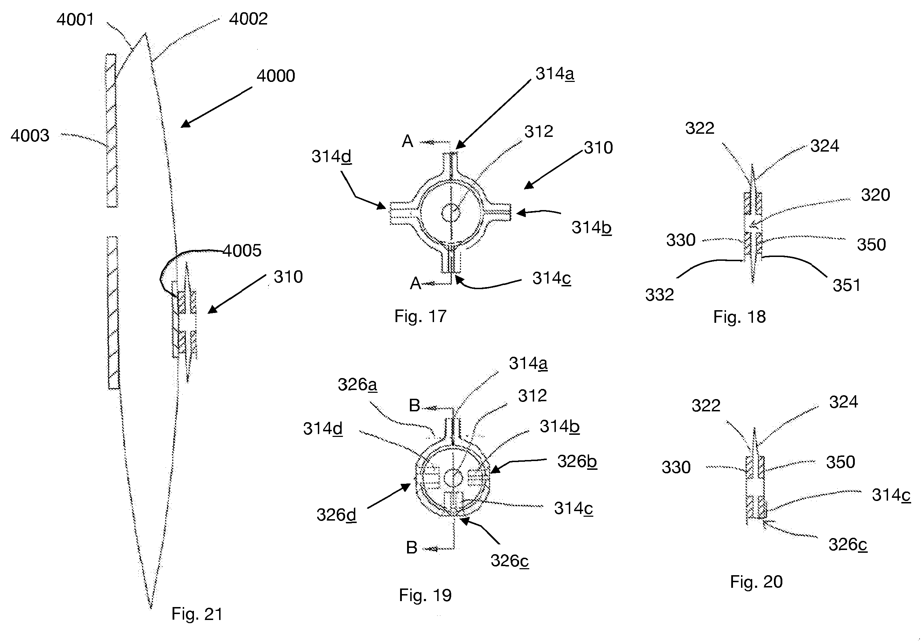

Referring now to figures 17 to 21 there is shown a fourth embodiment of a device 310 in accordance with the present invention. The device 310 includes a plurality of outlet 314a, 314b, 314c, 314d, each of which is in fluid communication with the inlet 312 of the device 310. In the present example the outlets 314a, 314b, 314c, 314d are provided as elongated gas flow path channels which extend away from the main gas flow path 320.

In the present example the cross sectional area of each of the outlets 314a, 314b, 314c, 314d, differ from each other. In the present example the outlet 314d has the largest cross sectional area. The outlet 314a has the smallest cross sectional area. The outlets 314b and 314c have cross sectional areas sized between the outlet 314a and outlet 314d. This means that a number of permutations of gas flow rate can be achieved through the device 310.

In a similar fashion to the third embodiment shown in figures 12 to 16, each of the plurality of outlets 314a, 314b, 314c, 314d can be closed in order for the user to select a desired rate of gas flow through the device 310. In order to achieve this an adhesive pad 350 is provided in order to hold one or all of the outlets in their folded, closed, configuration.

In order to close an outlet, the outlet is folded back on itself towards the adhesive pad 350 about its respective fold line 326a, 326b, 326c, 326d. Figure 19 shows the configuration where the outlets 314b, 314c, 314d have been closed. This means that gas can flow only from the inlet 312 to the outlet 314a.

It will be appreciated, of course, that any desired combination of the outlets 314a, 314b, 314c, 314d can be closed/open. It should also be appreciated that all four of the outlets can be closed thus preventing any flow of gas through the device 310.

In the above embodiments the adhesive pad 50, 150, 250, 350, may be configured such that it is a permanent adhesive or such that any part of the device adhered thereto can be removed, and later re-adhered, by a user.

## Claims

1. A gas flow regulation device (110) for connection to a gas outlet of an ostomy appliance, the device including:
an inlet (112) for communicating with the gas outlet of the ostomy appliance;
an outlet (114a, 114b, 114c) from which gas can exit the device,
means for closing the outlet from the device; and
a gas flow path (120) between the inlet and the outlet,
**characterised in that** said means includes folding the gas flow path back on itself to permit the rate of gas flow through the device to be user selectable.

2. A gas flow regulation device according to claim 1 wherein the device includes means (150) for holding the gas flow path in its folded configuration.

3. A gas flow regulation device according to claim 2 wherein the holding means includes an adhesive (150).

4. A gas flow regulation device according to any one of the preceding claims wherein the device includes a plurality of outlets (214a, 214b, 214c, 214d) from which gas can exit the device, each outlet having a respective gas flow path and being in fluid communication with the inlet.

5. A gas flow regulation device according to claim 4 wherein means is provided for closing one or more of the plurality of outlets.

6. A gas flow regulation device according to claim 5 wherein said means includes folding the respective gas flow path of one or more of the plurality of outlets.

7. A gas flow regulation device according to any one of claims 4 to 6 wherein the device includes means for holding each respective gas flow path in its folded configuration.

8. A gas flow regulation device according to any one of claims 4 to 7 wherein the cross-sectional area of each of the outlets is substantially the same.

9. A gas flow regulation device according to any one of claims 4 to 7 wherein the cross-sectional area of each outlet (314a, 314b, 314c, 314d) differs from the other outlets (314a, 314b, 314c, 314d).

10. A gas flow regulation device according to any one of claims 4 to 9 including three outlets, wherein:
a first outlet (314d) has the largest cross-sectional area;
a third outlet (314a) has the smallest cross-sectional area; and
a second outlet (314b, 314c) having a cross-sectional area sized between the first and third outlets.

11. A gas flow regulation device according to any one of claims 4 to 9 including four outlets (314a, 314b, 314c, 314d), wherein:
a first outlet (314d) has the largest cross-sectional area;
a fourth outlet (314a) has the smallest cross-sectional area;
a second outlet (314b) having a cross-sectional area sized between the first and fourth outlets; and
a third outlet (314c) having a cross-sectional area sized between the first and fourth outlets.

12. A gas flow regulation device according to any one of the preceding claims wherein the or each gas flow path is divided into a plurality of chambers (137, 138, 139) with each chamber including a respective outlet (114a, 114b, 114c) for gas, and preferably adjacent chambers are connected by an opening (118a, 118b, 118c) through which gas can flow from one chamber to another chamber.

13. A gas flow regulation device according to claim 12 wherein the chambers are in sequential gas communication with each other.

14. A gas flow regulation device according to claim 12 or 13, including one or more or all of:
(a) the opening between respective chambers being closable by a user to alter the rate of gas flow through the device, wherein the device preferably includes means for holding the outlet in its folded configuration;
(b) the opening between respective chambers being closable by folding the outlet about the opening, wherein the device preferably includes means for holding the outlet in its folded configuration;
(c) the outlet(s) for gas being an aperture, and/or the outlet(s) for gas being a vented area, preferably the vented area including a plurality of small apertures, preferably laser-cut apertures, for permitting gas to exit through the outlet or the vented area including a gas permeable membrane for permitting gas to exit through the outlet;
(d) a pair of walls which are connected to each other at or near their peripheries;
(e) means for coupling the inlet to a gas outlet of an ostomy appliance, preferably said means including an adhesive covered by a removable cover member;
(f) the outlet(s) being provided on an extension part which extends away from the inlet; and
(g) the outlet(s) is provided an extension part which extends radially away from the inlet.

15. An ostomy appliance including a device according to any preceding claim coupled to a gas outlet thereof.

## Patentansprüche

1. Gasdurchflussregelungsvorrichtung (110) zum Anschluss an einen Gasauslass eines Stomageräts, wobei das Gerät einschließt:
Einen Einlass (112) zum Kommunizieren mit dem Gasauslass des Stomageräts;
einen Auslass (114a, 114b, 114c) aus dem Gas aus dem Gerät
entweichen kann, Mittel zum Schließen des Auslasses ab dem
Gerät; und einen Gasdurchflussweg (120) zwischen dem
Einlass und dem Auslass,
**dadurch gekennzeichnet, dass** das genannte Mittel das Falten
des Gasdurchflusswegs auf sich zurück einschließt,
um Benutzer selektierbare Flussrate von Gas durch das Gerät zu gestatten.

2. Gasdurchflussregelungsvorrichtung nach Anspruch 1, wobei die Vorrichtung Mittel (150) zum Halten des Gasdurchflusswegs in seiner gefalteten Konfiguration einschließt.

3. Gasdurchflussregelungsvorrichtung nach Anspruch 2, wobei das Haltemittel einen Klebstoff (150) einschließt.

4. Gasdurchflussregelungsvorrichtung nach irgendeinem der vorhergehenden Ansprüche, wobei die Vorrichtung eine Vielzahl von Auslässen (214a, 214b, 214c, 214d) einschließt, aus denen Gas aus der Vorrichtung entweichen kann, jeder Auslass einen jeweiligen Gasdurchflussweg aufweist und in Fluidverbindung mit dem Einlass steht.

5. Gasdurchflussregelungsvorrichtung nach Anspruch 4, wobei das Mittel zum Schließen eines oder mehrerer der Vielzahl von Auslässen bereitgestellt ist.

6. Gasdurchflussregelungsvorrichtung nach Anspruch 5, wobei das genannte Mittel das Falten des jeweiligen Gasdurchflusswegs eines oder mehrerer der Vielzahl von Auslässen einschließt.

7. Gasdurchflussregelungsvorrichtung nach irgendeinem der Ansprüche 4 bis 6, wobei die Vorrichtung Mittel einschließt, um jeden jeweiligen Gasdurchflussweg in seiner gefalteten Konfiguration zu halten.

8. Gasdurchflussregelungsvorrichtung nach irgendeinem der Ansprüche 4 bis 7, wobei die Querschnittsfläche jedes der Auslässe im Wesentlichen gleich ist.

9. Gasdurchflussregelungsvorrichtung nach irgendeinem der Ansprüche 4 bis 7, wobei Querschnittsfläche jedes Auslasses (314a, 314b, 314c, 314d) von de4n anderen Auslässen (314a, 314b, 314c, 314d) verschieden ist.

10. Gasdurchflussregelungsvorrichtung nach irgendeinem der Ansprüche 4 bis 9, die drei Auslässe einschließt, wobei:
ein erster Auslass (314d) die größte Querschnittsfläche aufweist;
ein dritter Auslass (314a) die kleinste Querschnittsfläche aufweist; und ein zweiter Auslass (314b, 314c) eine Querschnittsfläche aufweist, die zwischen den ersten und dritten Auslässen bemessen ist.

11. Gasdurchflussregelungsvorrichtung nach irgendeinemder Ansprüche 4 bis 9, die vier Auslässe (314a, 314b, 314c, 314d) einschließt, wobei:
ein erster Auslass (314d) die größte Querschnittsfläche aufweist; ein vierter Auslass (314a) die kleinste Querschnittsfläche aufweist; ein zweiter Auslass (314b) eine Querschnittsfläche aufweist, die zwischen den ersten und vierten Auslässen bemessen ist; und
ein dritter Auslass (314c) eine Querschnittsfläche aufweist, die zwischen den ersten und vierten Auslässen bemessen ist.

12. Gasdurchflussregelungsvorrichtung nach irgendeinem der vorhergehenden Ansprüche, wobei der oder jeder Gasdurchflussweg in eine Vielzahl von Kammer (137, 138, 139) unterteil ist, wobei jede Kammer einen jeweiligen Auslass (114a, 114b, 114c) für Gas einschließt und vorzugsweise angrenzende Kammern durch eine Öffnung (118a, 118b, 118c) verbunden sind, durch die Gas von einer Kammer zu einer anderen Kammer strömen kann.

13. Gasdurchflussregelungsvorrichtung nach Anspruch 12, wobei die Kammern in sequenzieller Gasverbindung miteinander stehen.

14. Gasdurchflussregelungsvorrichtung nach Anspruch 12 oder 13, die eins oder mehrere oder alle der Folgenden einschließt:
(a) Der Öffnung zwischen jeweiligen Kammern, die von einem Benutzer schließbar sind, um die Gasdurchflussrate durch die Vorrichtung zu ändern, wobei die Vorrichtung vorzugsweise Mittel zum Halten des Auslasses in seiner gefalteten Konfiguration einschließt;
(b) die Öffnung zwischen jeweiligen Kammern durch Falten des Auslasses um die Öffnung schließbar ist, wobei die Vorrichtung vorzugsweise Mittel zum Halten des Auslasses in seiner gefalteten Konfiguration einschließt;
(c) der/die Auslass/Auslässe für Gas eine Öffnung sind, und/oder der/die Auslass/Auslässe für Gas ein entlüfteter Bereich ist/sind, der eine Vielzahl von kleinen Öffnungen, vorzugsweise von einem Laser geschnittene Öffnungen einschließt, um Gas durch den Auslass oder den entlüfteten Bereich entweichen zu lassen, der eine gasdurchlässige Membran einschließt, um Gas durch den Auslass entweichen zu lassen;
(d) ein Paar Wände, die an den oder nahe von deren Umfängen miteinander verbunden sind;
(e) Mitteln zum Koppeln des Einlasses an einen Gasauslass eines Stomageräts, vorzugsweise schließen die genannten Mittel einen von einem entfernbaren Deckelement abgedeckten Klebstoff ein;
(f) den/die Auslass/Auslässe, die auf einem Verlängerungsteil bereitgestellt sind, das sich weg vom Einlass erstreckt; und
(g) der/die Auslass/Auslässe an einem Verlängerungsteil bereitgestellt ist/sind, das sich radial weg vom Einlass erstreckt.

15. Stomagerät, das eine Vorrichtung nach irgendeinem vorhergehenden Anspruch einschließt, die an einen Gasauslass davon gekoppelt ist.

## Revendications

1. Un dispositif de régulation de flux de gaz (110) destiné à être relié à un orifice de sortie de gaz d'un accessoire pour stomie, le dispositif incluant :
un orifice d'entrée (112) pour communiquer avec l'orifice de sortie de gaz de l'accessoire pour stomie ;
un orifice de sortie (114a, 114b, 114c) par lequel le gaz peut sortir du dispositif,
un moyen de fermeture de l'orifice de sortie du dispositif ; et
une voie d'écoulement du gaz (120) entre l'orifice d'entrée et l'orifice de sortie,
**caractérisé en ce que** ledit moyen inclut le pliage de la voie d'écoulement du gaz sur elle-même pour permettre au débit du flux de gaz de passer dans le dispositif sélectionnable par l'utilisateur.

2. Un dispositif de régulation de flux de gaz selon la revendication 1 dans lequel le dispositif inclut un moyen (150) pour maintenir la voie d'écoulement du gaz à sa configuration repliée.

3. Un dispositif de régulation de flux de gaz selon la revendication 2 dans lequel le moyen de maintien inclut un adhésif (150).

4. Un dispositif de régulation de flux de gaz selon l'une quelconque des revendications précédentes dans lequel le dispositif inclut une pluralité d'orifices de sortie (214a, 214b, 214c, 214d) par lesquels le gaz peut sortir du dispositif, chaque orifice de sortie ayant respectivement une voie d'écoulement du gaz et étant en communication fluidique avec l'orifice d'entrée.

5. Un dispositif de régulation de flux de gaz selon la revendication 4 dans lequel un moyen est fourni pour fermer une ou plusieurs de la pluralité d'orifices de sortie.

6. Un dispositif de régulation de flux de gaz selon la revendication 5 dans lequel ledit moyen inclut le pliage de la voie d'écoulement du gaz respective d'une ou de plusieurs de la pluralité d'orifices de sortie.

7. Un dispositif de régulation de flux de gaz selon l'une quelconque des revendications 4 à 6 dans lequel le dispositif inclut un moyen pour maintenir chaque voie d'écoulement du gaz respective dans sa configuration repliée.

8. Un dispositif de régulation de flux de gaz selon l'une quelconque des revendications 4 à 7 dans lequel la section transversale de chacun des orifices de sortie est sensiblement la même.

9. Un dispositif de régulation de flux de gaz selon l'une quelconque des revendications 4 à 7 dans lequel la section transversale de chaque orifice de sortie (314a, 314b, 314c, 314d) diffère de celle des autres orifices de sortie (314a, 314b, 314c, 314d).

10. Un dispositif de régulation de flux de gaz selon l'une quelconque des revendications 4 à 9 incluant trois orifices de sortie, dans lequel :
un premier orifice de sortie (314d) a la section transversale la plus grande ;
un troisième orifice de sortie (314a) a la section transversale la plus petite ; et
un deuxième orifice de sortie (314b, 314c) a une section transversale dimensionnée entre celle des premier et troisième orifices de sortie.

11. Un dispositif de régulation de flux de gaz selon l'une quelconque des revendications 4 à 9 incluant quatre orifices de sortie (314a, 314b, 314c, 314d), dans lequel :
un premier orifice de sortie (314d) a la section transversale la plus grande ;
un quatrième orifice de sortie (314a) a la section transversale la plus petite ;
un deuxième orifice de sortie (314b) a une section transversale dimensionnée entre celle des premier et quatrième orifices de sortie ; et
un troisième orifice de sortie (314c) a une section transversale dimensionnée entre celle des premier et quatrième orifices de sortie.

12. Un dispositif de régulation de flux de gaz selon l'une quelconque des revendications précédentes dans lequel la ou chaque voie d'écoulement du gaz est divisée en une pluralité de chambres (137, 138, 139), chaque chambre incluant un orifice de sortie respectif (114a, 114b, 114c) pour le gaz, et de préférence les chambres adjacentes sont reliées par une ouverture (118a, 118b, 118c) par laquelle le gaz peut s'écouler d'une chambre dans un autre chambre.

13. Un dispositif de régulation de flux de gaz selon la revendication 12 dans lequel les chambres sont en communication gazeuse séquentielle les unes avec les autres.

14. Un dispositif de régulation de flux de gaz selon la revendication 12 ou 13, incluant une ou plusieurs ou toutes les caractéristiques constituées par :
(a) l'ouverture entre les chambres respectives étant fermable par un utilisateur pour modifier le débit du flux de gaz passant dans le dispositif, dans lequel le dispositif inclut de préférence un moyen pour maintenir l'orifice de sortie à sa configuration repliée ;
(b) l'ouverture entre les chambres respectives étant fermable en repliant l'orifice de sortie sur l'ouverture, dans lequel le dispositif inclut de préférence un moyen pour maintenir l'orifice de sortie à sa configuration repliée ;
(c) le ou les orifice(s) de sortie du gaz étant une ouverture, et/ou le ou les orifice(s) de sortie du gaz étant une zone de mise à l'air libre, la zone de mise à l'air libre incluant de préférence une pluralité de petites ouvertures, de préférence des ouvertures découpées au laser, pour permettre au gaz de sortir par l'orifice de sortie ou la zone de mise à l'air libre incluant une membrane perméable au gaz pour permettre au gaz de sortir par l'orifice de sortie ;
(d) une paire de parois qui sont reliées l'une à l'autre à ou proche de, leur périphérie ;
(e) un moyen pour raccorder l'orifice d'entrée à une sortie de gaz d'un accessoire pour stomie, de préférence ledit moyen incluant un adhésif couvert par un élément de couvercle amovible ;
(f) le ou les orifice(s) de sortie étant muni(s) d'une rallonge qui s'écarte de l'orifice d'entrée ; et
(g) le ou les orifice(s) de sortie est ou sont muni(s) d'une rallonge qui s'écarte radialement de l'orifice d'entrée.

15. Un accessoire pour stomie incluant un dispositif selon l'une quelconque des revendications précédentes, raccordé à un orifice de sortie de gaz de celui-ci.
